(19) Europäisches Patentamt
European Patent Office
Office européen des brevets

(11) Numéro de publication: **0 102 271**
**B1**

(12) # FASCICULE DE BREVET EUROPEEN

(45) Date de publication du fascicule du brevet: **08.01.86**

(51) Int. Cl.[4]: **C 07 C 119/08**

(21) Numéro de dépôt: **83401529.9**

(22) Date de dépôt: **26.07.83**

(54) **Procédé de stabilisation d'imines réactives, sels d'iminium stables obtenus par ce procédé et application de ces sels.**

(30) Priorité: **29.07.82 FR 8213273**

(43) Date de publication de la demande: **07.03.84 Bulletin 84/10**

(45) Mention de la délivrance du brevet: **08.01.86 Bulletin 86/2**

(84) Etats contractants désignés: **BE CH DE GB IT LI LU NL**

(56) Documents cités:
**US - A - 2 278 163**

**CHEMICAL ABSTRACTS, vol. 87, no. 25, 19 décembre 1977, page 690, no. 201135t, Columbus, Ohio, USA**

(73) Titulaire: **CENTRE NATIONAL DE LA RECHERCHE SCIENTIFIQUE (C.N.R.S.), 15, Quai Anatole France, F-75007 Paris (FR)**

(72) Inventeur: **Denis, Jean-Marc, 56, rue Thiers, F-59650 Villeneuve d'Ascq (FR)**
Inventeur: **Guillemin, Jean-Claude, 43, rue de Heilles, F-60250 Mouy (FR)**
Inventeur: **Lablache-Combier, Alain, 64, Avenue du Vieux Château, F-59650 Villeneuve d'Ascq (FR)**

(74) Mandataire: **Roger-Petit, Jean-Camille et al, OFFICE BLETRY 2, Boulevard de Strasbourg, F-75010 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 102 271 B1

## Description

Il est connu de préparer des molécules très réactives et à l'état très pur, et notamment des imines, en particulier dont les squelettes moléculaires sont les plus simples, par déshydrohalogénation d'halogénures d'amines précurseurs; cette déshydrohalogénation est effectuée en phase vapeur sous vide, sur une base hydroxylique solide, forte et non nucléophile, telle que le tertio-butylate de potassium ou l'adamantylate de potassium, éventuellement incorporée à un support solide, tel que la silice ou l'alumine (voir J.M. Denis *et al.*, «J. Amer. Chem. Soc.», 1981, 103, p. 468; J.M. Denis *et al.*, «Nouveau Journal de Chimie», vol. 6, N° 3, 1982, p. 121; J.C. Guillemin, «Thèse de 3ᵉ cycle», N° d'ordre 970, soutenue à l'Université des Sciences Techniques de Lille, le 23 juin 1982).

Ces imines très réactives, et notamment celles ayant les formules ci-dessous, qui n'ont pu être obtenues jusqu'alors que par le procédé sus-indiqué, sont instables. Or, elles sont d'un intérêt synthétique considérable, car ce sont les systèmes de base les plus simples de la chimie organique.

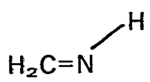
1

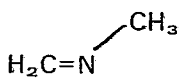
2

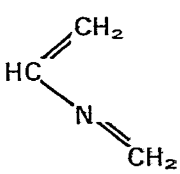
3

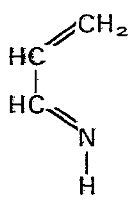
4

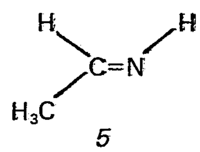
5

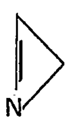
6

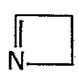
7

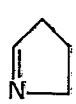
8

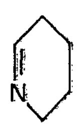
9

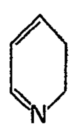
10

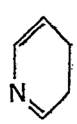
11

Toutes ces imines ont été étudiées à basse température (−100° C pour éviter leur oligomérisation) (IR, RMN, etc.).

La réactivité de ces imines est si grande qu'en général elles se polymérisent au réchauffement avant de réagir avec le réactif choisi.

La présente invention a pour premier objet un procédé de stabilisation de ces imines réactives par leur transformation en sels d'iminium, qui soient parfaitement stables à température ordinaire ou à des températures de l'ordre de 50° C, de telle sorte que ces sels soient des produits stockables et commercialisables et par conséquent utilisables à titre de synthons, c'est-à-dire à titre de molécules clés de synthèse à la fois réactives et stables.

D'une façon générale, les sels d'iminium présentent une très grande réactivité vis-à-vis des nucléophiles et se prêtent à des réactions de cycloaddition. Ils occupent donc une place importante en synthèse organique, compte tenu de leur réactivité exceptionnelle, qui est liée au fait qu'une de leurs contributions mésomères peut être représentée sous la forme d'un ion carbénium stabilisé par une fonction amine:

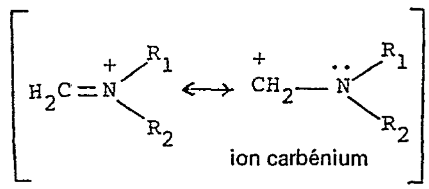
ion carbénium

Or, les sels d'iminium connus et stables sont essentiellement des sels disubstitués sur l'azote, et notamment le sel de Potier [P. Potier *et al.*, «J.A.C.S.», *90* (1968) 5622] et le sel d'Eschenmoser [A. Eschenmoser *et al.*, «Angew. Chem. Int.», éd. *10* (1971) 330]:

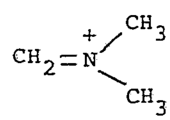
$CF_3COO^{\ominus}$
Sel de Potier

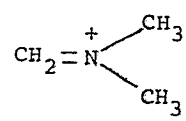
$I^{\ominus}$
Sel d'Eschenmoser

Ces deux sels ont de nombreuses applications synthétiques.

Toutefois, les sels d'iminium monosubstitués sur l'azote ou les composés parents (non substitués sur l'azote), répondant aux formules schématiques ci-après:

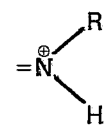
$X^{\ominus}$

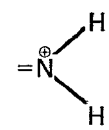
$X^{\ominus}$

R représentant un substituant et X un anion sont pratiquement inconnus, n'ayant jamais pu être isolés.

Ces sels d'iminium peuvent cependant être considérés comme des synthons d'une haute potentialité, puisqu'ils sont susceptibles d'introduire, dans les différentes additions avec des nucléophiles, une amine secondaire ou primaire très utile pour des applications synthétiques ultérieures, ladite amine pouvant être fonctionnalisée, alors que le sel de Potier ou le sel d'Eschenmoser permettent uniquement d'introduire une amine tertiaire.

Les demandeurs ont d'abord tenté de former un sel d'iminium monosubstitué sur l'azote en protonant une imine simple, à savoir la N-méthylméthanimine (formule 2 ci-dessus), au moyen de l'acide chlorhydrique ou de l'acide trifluoroacétique. Les sels d'iminium obtenus sont peu stables et très hygroscopiques et ils ne peuvent être

purifiés et utilisés aisément en synthèse organique. La réaction avait été effectuée par addition de l'iminium à basse température (inférieure à −60° C) dans une solution saturée de l'acide. On a dû constater que la formation de sels d'iminium par réaction entre l'imine et un acide en solution ne constitue pas une méthode intéressante de stabilisation des imines. Le rendement de la réaction est en outre très faible.

C'est alors que les demandeurs ont découvert le présent procédé de stabilisation d'imines réactives par transformation de ces imines en sels d'iminium, ce procédé étant caractérisé en ce que l'on effectue cette transformation par une réaction d'addition de l'imine à l'état dilué, sur un acide fort solide de grande surface spécifique, choisi parmi les acides polybenzènesulfoniques, la réaction, qui conduit aux sels d'iminium monosubstitués ou non substitués sur l'azote des formules sus-indiquées, où R représente un substituant et X un anion d'acide polybenzènesulfonique, étant obtenue par passage de l'imine en phase vapeur ou gazeuse, sous vide, à la température ordinaire, sur l'acide fort solide, ou par mise en contact de l'imine, à basse température, sous la forme d'une solution diluée dans un solvant organique de bas point de fusion, avec l'acide fort solide, et lesdites imines réactives, qui répondent aux formules schématiques ci-après:

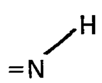

et

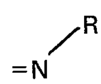

où R représente un substituant, étant choisies parmi les composés des formules 1 à 11 ci-dessus.

Suivant la première méthode, un vide classique, de l'ordre de $10^{-1}$ à $10^{-2}$ mm de mercure peut suffire et, suivant la seconde méthode, la température basse peut être de l'ordre de −80° C.

On a cherché à éviter toute réaction intermoléculaire entre deux ou plusieurs molécules d'une même espèce et en particulier à empêcher l'oligomérisation de l'imine. C'est pourquoi l'imine doit être diluée. L'acide doit être fort, l'imine étant une base faible.

Les imines mises en œuvre, qui sont préparées par le procédé sus-indiqué, sont essentiellement des imines volatiles, correspondant aux formules 1 à 11 ci-dessus, ces imines devant de préférence avoir une tension de vapeur suffisante pour se volatiliser sous le vide classique sus-indiqué, qui est par exemple celui de la pompe à palette.

La volatilité est moins nécessaire lorsque l'imine intervient en solution, bien qu'il soit commode en pratique d'effectuer le mélange de l'imine et du solvant organique à l'état de vapeurs sous vide. Le solvant organique de bas point de fusion employé selon ce deuxième mode opératoire peut être l'éther, le chloroforme, le tétrahydrofuranne, le chlorure de méthylène, le mélange $CH_2Cl_2-CFCl_3$, ou des mélanges de ces solvants, dont la liste n'est pas limitative.

L'acide fort solide utilisé comme agent de protonation de l'imine est avantageusement sous forme de grains poreux ou de poudre, afin d'offrir une grande surface spécifique pour l'addition de l'imine. On emploie des résines sulfonées, c'est-à-dire des polymères dans lesquels les sites acides sont dispersés. Ce sont des acides polybenzènesulfoniques, dont la formule schématique est la suivante:

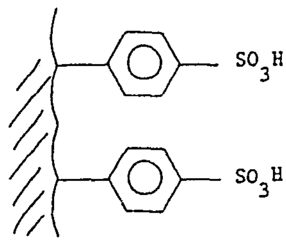

par simplification

Ⓟ—$SO_3H$

On peut mentionner en particulier les résines échangeuses d'ions Amberlite et Amberlyst 15 de la société Röhm and Haas, qui sont des copolymères sulfonés de styrène et de divinylbenzène, les Sphérosils acides de la société Rhône-Poulenc et le Nafion-H, qui est une résine sulfonée et perfluorée fabriquée par la société américaine DuPont de Nemours. L'imine se fixe sur les sites acides en engendrant le sel d'iminium. La teneur desdites résines en équivalents d'ion hydrogène par gramme est connue, ce qui permet d'effectuer la réaction avec l'imine dans des proportions stœchiométriques ou dans tout autre rapport désiré. Ces résines, qui sont des réactifs commerciaux, sont avantageuses pour une fabrication industrielle de sels d'iminium.

L'imine mise en œuvre résultant de la déshydrohalogénation de la chloramine correspondante et étant préparée en phase vapeur sous vide, comme sus-indiqué, il est inutile d'isoler intermédiairement l'imine obtenue, que l'on fait passer dans la même ligne de vide sur l'acide solide, ou que l'on mélange sous vide avec un solvant volatil, le mélange étant ensuite refroidi pour être recueilli à l'état liquide dans un collecteur, où il est mis en contact avec l'acide fort solide.

Le produit final est constitué par cet acide fort solide sur lequel l'imine s'est fixée. Ce produit peut être conservé sans altération très longtemps en l'absence d'air, par exemple sous azote.

La présente technique de synthèse d'imines réactives sur des bases hydroxyliques solides et de synthèse subséquente de sels d'iminium par réaction de ces imines avec des acides forts solides permet d'obtenir les sels d'iminium les plus simples, inconnus jusqu'alors, et notamment les suivants de formules 12 et 13, qui correspondent aux imines 1 et 2:

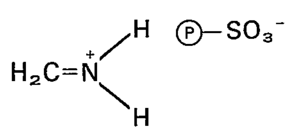

12

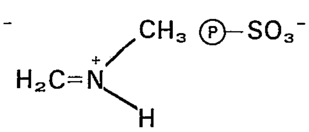

13

L'invention a également pour objet les sels d'iminium stables ainsi obtenus, caractérisés en ce qu'ils sont des sels d'iminium monosubstitués ou non substitués sur l'azote de formule

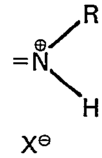

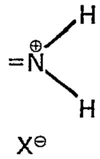

où R représente un substituant et X un anion d'acide polybenzènesulfonique ces sels d'iminium étant des composés d'addition sur un acide fort solide, choisi parmi les acides polybenzènesulfoniques, d'une imine réactive choisie parmi les imines simples 1 à 11 sus-indiquées.

Un autre objet de l'invention est l'application de ces sels d'iminium, en tant que précurseurs de synthèses commercialement accessibles, à la fabrication de composés très élaborés, inconnus jusque alors et de haute potentialité.

Toutes les réactions effectuées actuellement avec les sels de Potier ou les sels d'Eschenmoser sont applicables aux présents sels d'iminium, en particulier la réaction de Mannich, la réaction de Diels-Alder, la réaction de Leuckart-Wallach, la réaction de Polonovski, la méthylation d'Eschweiller-Clarke, la synthèse de Strecker, la fragmentation des γ-halogénoalkylamines, etc. Les produits ainsi obtenus, qui résultent de réactions avec des nucléophiles, peuvent être des intermédiaires de synthèse d'alcaloïdes. Les présents sels d'iminium permettent aussi la synthèse rapide d'alcaloïdes hétérocycliques de type quinuclidine ou isoquinuclidine par réaction de cycloaddition hétérodiénique.

Le schéma 1 ci-après donne des exemples d'addition de nucléophiles, le schéma 2 un exemple de cycloaddition de type [4 + 2] et le schéma 3 un autre exemple de cycloaddition de type [4 + 2] entre la dihydro-3,4 pyridine (composé 11) et une oléfine activée, fournissant une isoquinuclidine insaturée:

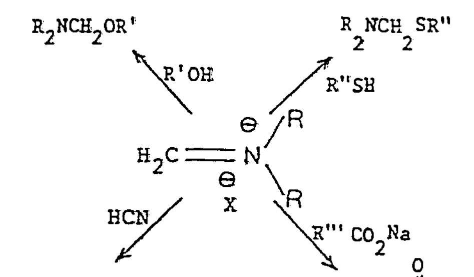


Schéma 1: (addition de nucléophiles)

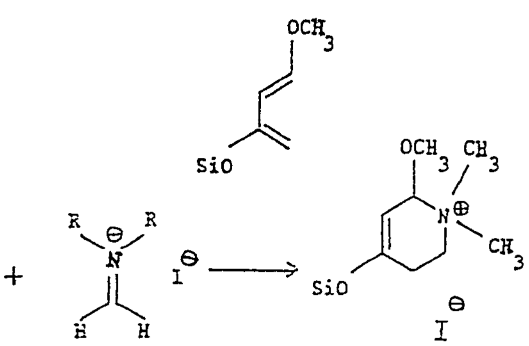

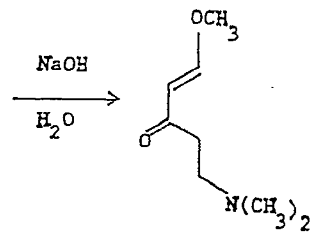


Schéma 2: (cycloaddition [4 + 2])

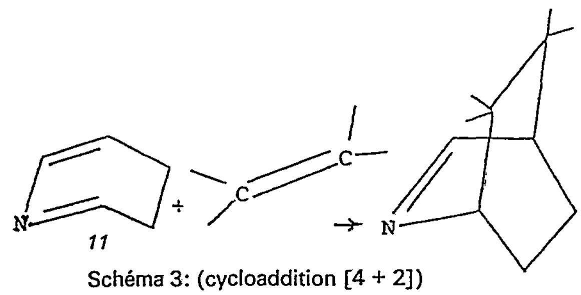

11

Schéma 3: (cycloaddition [4 + 2])

Dans les exemples ci-après, on décrit des modes particuliers de mise en œuvre du procédé suivant l'invention, en référence au dessin annexé, qui est une représentation schématique de l'appareillage utilisé.

*Exemple 1:*

*Synthèse du sel de N-méthylméthaniminium de la formule 13 sus-indiquée*

On utilise comme matière première 27 la N-chlorodiméthylamine de formule $(CH_3)_2N-Cl$ (9,15 g, $10^{-1}$ mol), placée dans un récipient 1, en vue de réagir avec une base hydroxylique solide 2, qui est du tertio-butylate de potassium (34 g, 0,3 mol), placé dans le tube interne 3, en Pyrex par exemple, d'un four tournant 4. Le tube en Pyrex 3 est rempli de la base solide jusqu'à demi-section, cette base étant maintenue dans la région centrale du tube entre deux tampons de laine de verre 28 et 29. Le tube 3 est monté rotatif en 5 et en 6, au moyen de roulements à billes et de joints toriques assurant l'étanchéité entre une canalisation 7 de liaison au récipient 1 et une canalisation de vide 8. Il peut être entraîné de façon à tourner lentement avec le four thermostatique 4 susceptible de chauffer à une température de 20 à 250° C et réglé à une température de 55 à 60° C pour la réaction présentement considérée. La rotation permet de renouveler les sites actifs du réactif solide. Le vide est assuré à l'extrémité de la canalisation ou ligne de vide 8 par une pompe à palette (débit 35 $m^3$). Le vide réalisé est de l'ordre de $10^{-1}$ à $10^{-2}$ mm de mercure; il provoque la vaporisation de la diméthylchloramine vers le four tournant 4. L'entraînement mécanique du four tournant n'est pas représenté. Une vanne à pointeau en polytétrafluoréthylène schématisée en 9 permet de régler l'introduction de la substance de départ 1. Une jauge Pirani non représentée peut être branchée en amont ou en aval du four pour mesurer la pression. Le tertiobutylate de potassium 2 est avantageusement mélangé à un support solide tel que silice ou alumine.

Sur le banc de base solide 2, la diméthylchloramine mise en œuvre est déshydrohalogénée; il résulte de la réaction du chlorure de potassium solide, qui demeure dans le four, et un mélange de vapeurs de N-méthylméthanimine et de butanol tertiaire, qui s'échappe du four tournant 4 en 10 pour pénétrer dans un piège en U 11 maintenu à une température de −80° C et qui permet de séparer par condensation en 12 le butanol tertiaire.

L'imine, toujours à l'état de vapeur, qui s'échappe en 33, traverse ensuite un banc de protonation 13, sous forme d'un tube en Pyrex comparable au tube 3, également monté rotatif sur la ligne de vide par l'intermédiaire de roulements à billes et de joints d'étanchéité toriques 14 et 15. Ce banc de protonation est rempli jusqu'à demi-section et entre deux tampons de laine de verre d'un acide fort solide 16, tel que l'Amberlyst 15 de la société Röhm et Haas, qui est un acide polybenzène sulfonique sous forme de petites billes poreuses dites macroréticulées d'un diamètre compris entre environ 0,8 mm et environ 0,3 mm. Le banc de protonation 13, tout comme le four tournant 4, est entraîné en rotation lente, par un dispositif mécanique non représenté, afin de renouveler les sites actifs de l'acide solide durant le passage de la vapeur d'imine, ceci afin d'optimiser le rendement de la réaction d'addition. On utilise un équivalent de sites acides pour un équivalent d'imine; le banc de protonation contient en conséquence 21,2 g de l'acide solide, soit $10^{-1}$ équivalent molaire de sites acides.

On introduit dans l'appareillage la diméthylchloramine par vaporisation sous vide pendant 1 h.

Le sel d'iminium formé est retenu dans le banc de protonation, tandis que l'imine n'ayant pas réagi et s'échappant en 17 est condensée dans l'enceinte interne 18 d'un vase de Dewar, dont l'enceinte externe 19 est un réservoir d'azote liquide 20. Pour éviter la condensation d'éventuels produits lourds dans le tube interne 21 du Dewar, le flux gazeux traversant ce tube est maintenu à environ 25° C jusqu'à la base de celui-ci par une circulation de fluide chauffant dans l'enceinte 22.

Lorsque la réaction est considérée terminée, des vannes 23 et 24 placées sur la ligne de vide de part et d'autre du Dewar sont fermées et le Dewar est ramené à la pression ordinaire par l'introduction d'un gaz inerte tel que de l'azote en 25. Il s'ensuit un réchauffement qui provoque l'écoulement rapide du liquide déposé sur la paroi de l'enceinte interne 18 vers un collecteur 26 en liaison avec le fond du Dewar et refroidi à l'azote liquide. L'imine n'ayant pas réagi est ainsi recueillie à l'état condensé et peut être recyclée dans le dispositif.

Par ouverture de la vanne 23 et en continuant d'introduire du gaz neutre en 25, on met également sous plein d'azote le banc de protonation et l'acide fort solide renfermant le sel d'iminium formé par addition est alors introduit sous azote dans un récipient qui, s'il est bien hermétique, permet de conserver longtemps le sel formé.

Le rendement en sel d'iminium est de 30%, lorsqu'on met en œuvre, comme sus-indiqué, un équivalent de sites acides pour un équivalent d'imine (rapport 1/1). Le rendement est de 80% par rapport à l'acide, lorsqu'on utilise 5 Eq d'imine pour 1 Eq de sites acides.

Les rendements sont déterminés par l'augmentation de la masse de l'Amberlyst 15, par la masse des produits collectés en 12 et en 26, et ils sont confirmés par la quantité d'adduit formée après réaction du sel d'iminium sur un nucléophile (voir ci-dessous exemples 3 et 4).

On peut aussi augmenter le rendement en recyclant l'imine n'ayant pas réagi, recueillie en 26. En effet, l'imine passant à l'état de vapeur sur le banc d'acide fort solide, son temps de passage est relativement court, de l'ordre de la seconde, et tous les sites acides ne sont pas touchés, ce qui explique que le rendement soit d'environ 30% avec un seul passage de l'imine; avec trois passages de l'imine recueillie successivement, le rendement peut atteindre 80%.

Ces rendements s'entendent en poids du sel d'iminium obtenu par rapport au poids de l'imine mise en œuvre, c'est-à-dire de l'imine formée par réaction dans le four tournant 4.

Le sel d'iminium est obtenu sous une forme très pure, du fait que seule l'imine est fixée par l'acide solide et que cette imine, telle qu'elle est obtenue par le procédé décrit ci-dessus, est elle-même très pure.

*Exemple 2:*

*Synthèse du sel de méthaniminium de la formule 12 sus-indiquée*

On opère comme dans l'exemple 1 en partant de la N-chlorométhylamine (6,5 g, $10^{-1}$ mol). On utilise les mêmes quantités que dans l'exemple précédent, en ce qui concerne le tertio-butylate de potassium et l'Amberlyst 15.

On opère dans le four tournant à une température d'environ 50° C.

Les rendements en sel d'iminium sont les mêmes que ceux indiqués dans l'exemple précédent.

*Exemple 3:*

*Synthèse de la N-méthylpropanethio-2 méthylamine 15 (par addition du nucléophile propanethiol sur le sel de N-méthylméthaniminium 13)*

L'addition de propanethiol, nucléophile puissant, conduit à l'adduit attendu, d'où l'amine est facilement libérée par barbotage d'ammoniac.

La réaction est la suivante:

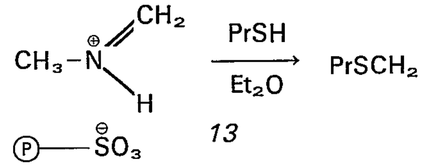


13

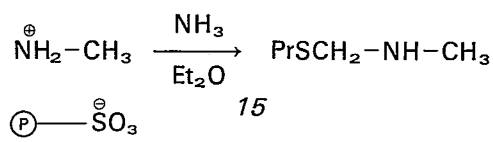


15

Pr représente un groupe propyle et Et un groupe éthyle.

Le sel d'iminium de la N-méthylméthanimine 13 (22,1 g d'Amberlyst $15{,}3 \cdot 10^{-2}$ Eq molaire du sel d'iminium 13) est introduit sous azote dans un ballon de 100 $cm^3$ muni d'un dessiccateur et d'une entrée d'azote. Le propanethiol (3,0 g, $4 \cdot 10^{-2}$ mol) dans 20 $cm^3$ d'éther éthylique anhydre est ajouté goutte à goutte pendant 15 min et la solution est encore agitée 10 min après la fin de l'addition. L'ensemble est filtré, le solide lavé plusieurs fois avec de l'éther éthylique et amené à sec à la pompe à palette. 30 $cm^3$ d'éther éthylique sont alors ajoutés. L'ensemble est refroidi à 0° C et agité mécaniquement; un barbotage d'ammoniac est effectué jusqu'à pH 11. La solution est filtrée et le solvant est évaporé à l'évaporateur rotatif; on obtient la N-méthylpropanethio-2 méthylamine 15 (3,42 g, $29 \cdot 10^{-3}$ mol) caractérisée par RMN du proton et masse.

Sauf lorsqu'on fait le vide, toutes les opérations sont effectuées sous azote ou autre gaz neutre.

RMN $^1H(CDCl_3)$
δ(ppm) 0,38(t, 3H, J = 3,3 Hz), 1,61 (m, 2H), 2,4 (s, 3H); 3,4 (t, 2H, J = 6,8 Hz), 4,04 (s, 2H).
Masse $M^+$ 119
m/e 118 (38, $(M-1)^{+\cdot}$), 89(13), 76(11), 75(100), 58(29), 47(66), 46(18), 45(36), 44(27), 43(40), 42(37), 41(28).
Rendement: 96%.

L'addition nucléophile et le déblocage de l'amine sont effectués avec un rendement pratiquement quantitatif et le produit obtenu n'avait jamais encore été synthétisé jusqu'alors.

*Exemple 4:*

*Synthèse de la propanethio-2 méthylamine 14 (par addition du nucléophile propanethiol sur le sel de méthaniminium 12)*

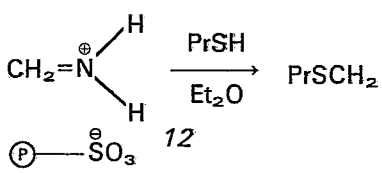


12

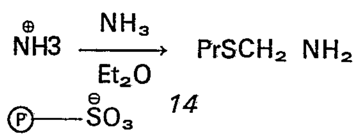


14

On opère comme dans l'exemple 3 à partir du sel d'iminium de la méthanimine 12 (22,1 g d'Amberlyst 15 saturé à 30% par $3 \cdot 10^{-2}$ mol du sel d'iminium 12). On obtient la propanethio-2 méthylamine 14 (3,18 g, $29 \cdot 10^{-3}$ mol) caractérisée par RMN et masse. Sa structure est confirmée par comparaison du spectre de RMN avec celui de l'éthanethio-2 méthylamine décrit dans la littérature [B. Braillon *et al.*, «Nouv. J. Chem.», *6* (1982) 121].

RMN $^1H(CDCl_3)$
δ(ppm) 0,98 (t, 3H, J = 7,3 Hz), 1,2 (s, 2H), 1,61 (m, 2H), 3,4 (t, 2H, J = 6,8 Hz), 4,1 (s, 2H).
Masse $M^+$ 91
m/e $90(M-1)^{+\cdot}$
Rendement: 95%.

Dans ce cas également, le rendement est pratiquement quantitatif et le produit obtenu est nouveau.

*Exemple 5:*

*Synthèse du sel de N-méthylméthaniminium de la formule 13 sus-indiquée*

On utilise le même appareillage que dans l'exemple 1, mais sans le banc de protonation 13. L'acide fort solide Amberlyst 15 est placé dans le collecteur 26 et un réservoir 30 d'éther éthylique 31 ou d'un solvant organique volatil similaire est branché sur la canalisation de vide en amont de la vanne 23. Une vanne à pointeau en polytétrafluoroéthylène 32 permet de régler l'introduction du solvant organique dans la canalisation de vide. Le solvant doit être anhydre.

On opère avec les mêmes quantités de N-chlorodiméthylamine, de tertio-butanolate de potassium et d'acide fort solide que dans l'exemple 1 et on procède comme dans cet exemple, sauf que la vanne à pointeau 32 étant ouverte, du solvant organique est introduit à l'état vaporisé dans la canalisation de vide, où il se mélange avec la vapeur d'imine sortant du piège en U 11. Le mélange d'imine et de solvant est condensé dans l'enceinte interne 18 du vase Dewar sous forme d'une solution d'imine dans le solvant.

La quantité d'éther éthylique anhydre à utiliser pour dissoudre l'imine formée est d'environ 2 $cm^3$ d'éther pour 85 mg de N-méthylméthanimine.

Lorsque la réaction engendrant l'imine est achevée, les vannes 23 et 24 sont fermées et le Dewar est ramené à la pression ordinaire par l'introduction d'un gaz neutre tel que de l'azote en 25. La solution d'imine dans le solvant organique s'écoule alors dans le collecteur 26 contenant l'acide fort solide, maintenu à une température d'environ −80° C et agité au moyen d'un agitateur magnétique. On laisse en contact le temps nécessaire pour que la réaction d'addition de l'imine sur l'acide se produise. Ce temps, relativement long du fait de la basse température qui diminue la réactivité, est de l'ordre de l'heure.

Lorsque la réaction d'addition de l'imine sur l'acide fort solide est terminée, on laisse réchauffer l'ensemble à température ordinaire, on filtre le contenu du collecteur 26 sous azote et on évapore le filtrat à la pompe à palette pour éliminer le solvant. On recueille et conserve sous azote le produit obtenu.

Le rendement est de l'ordre de 40% par rapport à l'imine; on ne peut éviter la formation de polymères de l'imine.

*Exemple 6:*

*Synthèse de la tétrahydro-1,2,5,6 triméthyl-1,3,4 pyridine 16*

Une réaction de cycloaddition [4 + 2] entre le sel d'iminium 13 et le diméthylbutadiène est effectuée (réaction de Diels-Alder).

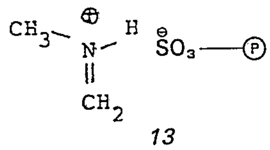

13

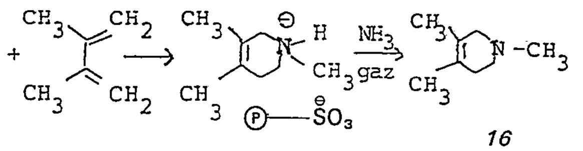


16

Le sel de N-méthylméthaniminium 13 préparé selon l'exemple 1 (20 g d'Amberlyst 15 neutralisé à 30% par l'imine 2, soit $28{,}2 \cdot 10^{-3}$ Eq de sel d'iminium) est introduit sous azote dans un ballon contenant 20 ml de chlorure de méthylène sec. On introduit ensuite sous azote à 20° C le diméthylbutadiène (2,46 g, soit $30 \cdot 10^{-3}$ mol). Après 48 h sous agitation à la température de la pièce, le mélange hétérogène est neutralisé par un courant d'ammoniac sec. On recueille, après évaporation du solvant et distillation, 2,75 g de tétrahydro-1,2,5,6 triméthyl-1,3,4 pyridine 16 (Rendement 77% par rapport au sel 13).

Le produit 16 est identifié par comparaison de ses spectres de masse, IR et de RMN avec un échantillon du même produit obtenu par synthèse univoque (E.M. Fry et E.L. May, «J. Org. Chem.», *26*, 1961, p. 2592).

A titre de variante dans l'exemple 5, on peut juste recouvrir de solvant organique anhydre l'acide fort solide placé dans le collecteur 26.

## Revendications

1. Procédé de stabilisation d'imines réactives par transformation de ces imines en sels d'iminium, caractérisé en ce que l'on effectue cette transformation par une réaction d'addition de l'imine à l'état dilué sur un acide fort solide de grande surface spécifique choisi parmi les acides polybenzènesulfoniques, la réaction, qui conduit aux sels d'iminium monosubstitués ou non substitués sur l'azote de formules

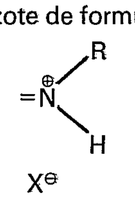 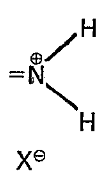

où R représente un substituant et X un anion d'acide polybenzènesulfonique, étant obtenue par passage de l'image en phase vapeur ou gazeuse, sous vide, à la température ordinaire, sur l'acide fort solide, ou par mise en contact de l'imine, à basse température, sous la forme d'une solution diluée dans un solvant organique de bas point de fusion, avec l'acide fort solide, et lesdites imines réactives, qui répondent aux formules schématiques ci-après:

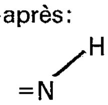 et 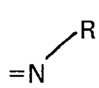

où R représente un substituant, étant choisies parmi les composés des formules 1 à 11 ci-après:

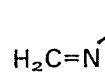 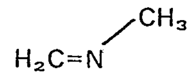

1 2

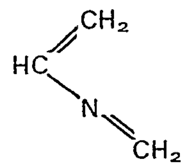 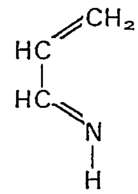 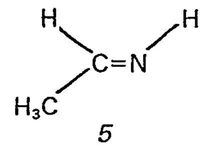

5

3 4

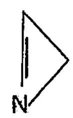 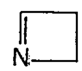 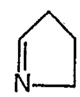 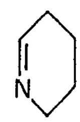

6 7 8 9

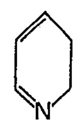 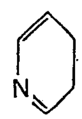

10 11

2. Procédé suivant la revendication 1, caractérisé en ce que l'acide fort est sous forme de grains poreux ou de poudre.

3. Procédé suivant l'une des revendications 1 ou 2, caractérisé en ce que l'on opère à −80° C, lorsque l'on fait réagir l'imine, à basse température, sous forme d'une solution diluée.

4. Procédé suivant l'une des revendications 1 à 3, caractérisé en ce que l'imine mise en œuvre est obtenue par déshydrohalogénation de la chloramine correspondante et est directement transformée, sans isolement intermédiaire, en le sel d'iminium recherché.

5. Sels d'iminium stables tels qu'obtenus par le procédé suivant l'une des revendications 1 à 4, caractérisés en ce qu'ils sont des sels d'iminium monosubstitués ou non substitués sur l'azote de formule

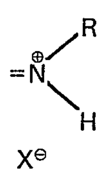 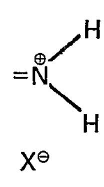

où R représente un substituant et X un anion d'acide polybenzènesulfonique, ces sels d'iminium étant des composés d'addition sur un acide fort solide, choisi parmi les acides polybenzènesulfoniques, d'une imine réactive choisie parmi les imines simples suivantes:

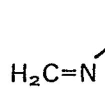 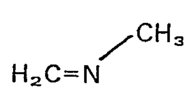

1 2

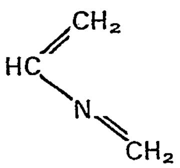 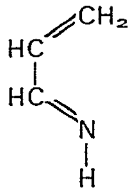 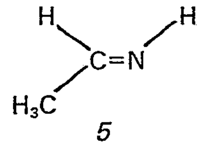

3 4 5

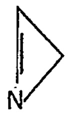 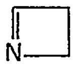 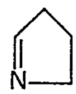 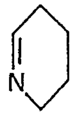

6 7 8 9

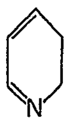 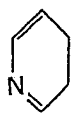

10 11

6. A titre de sels d'iminium stables suivant la revendication 5, le polybenzènesulfonate de N-méthylméthaniminium et le polybenzènesulfonate de méthaniminium.

7. Application des sels d'iminium suivant l'une des revendications 5 ou 6, caractérisée en ce que l'on soumet ces sels d'iminium, qui sont des synthons, à des réactions d'addition avec des nucléophiles ou à des réactions de cycloaddition avec des composés oléfiniques, pour obtenir des intermédiaires de synthèse d'alcaloïdes et des composés hétérocycliques tels que des alcaloïdes de type quinuclidine ou isoquinuclidine.

## Patentansprüche

1. Verfahren zur Stabilisierung von reaktiven Iminen durch Umwandlung dieser Imine in Iminiumsalze, dadurch gekennzeichnet, dass man diese Umwandlung bewirkt durch eine Additionsreaktion des Imins im verdünnten Zustand mit einer festen, starken Säure mit grosser spezifischer Oberfläche, ausgewählt unter den Polybenzolsulfonsäuren, wobei die Reaktion, die zu am Stickstoff monosubstituierten oder nicht substituierten Iminiumsalzen der Formeln

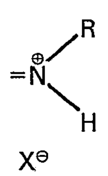 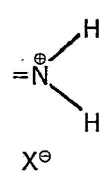

worin R einen Substituenten und X ein Anion von Polybenzolsulfonsäure darstellen, führt, durch Passage des Imins in Dampf- oder Gasphase unter Vakuum bei Normaltemperatur über die feste, starke Säure, oder durch Kontaktierung des Imins bei tiefer Temperatur in Form einer verdünnten Lösung in einem organischen Lösungsmittel mit tiefem Schmelzpunkt mit der festen, starken Säure bewirkt wird, und die besagten reaktiven Imine, die den nachstehenden schematischen Formeln entsprechen

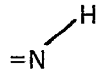 und 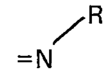

in welchen R einen Substituenten darstellt, ausgewählt sind unter den Verbindungen der nachstehenden Formeln 1 bis 11:

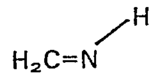 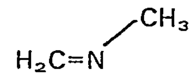

1 2

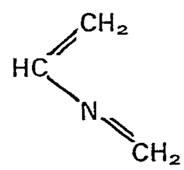 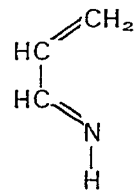 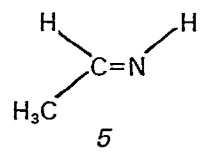

3 4 5

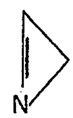 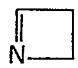 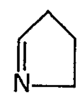 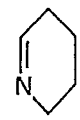

6 7 8 9

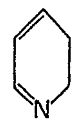 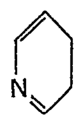

10 11

2. Verfahren gemäss Anspruch 1, dadurch gekennzeichnet, dass die starke Säure in Form poröser Körner oder eines Pulvers vorliegt.

3. Verfahren gemäss Anspruch 1 oder 2, dadurch gekennzeichnet, dass man bei −80° C arbeitet, wenn man das Imin bei tiefer Temperatur in Form einer verdünnten Lösung zur Umsetzung bringt.

4. Verfahren nach einem der Ansprüche 1 bis 3, dadurch gekennzeichnet, dass das eingesetzte Imin erhalten ist durch Deshydrohalogenierung des entsprechenden Chloramins und direkt umgewandelt wird ohne Zwischenisolierug in das herzustellende Iminiumsalz.

5. Stabile Iminiumsalze, wie sie erhalten werden nach dem Verfahren gemäss einem der Ansprüche 1 bis 4, dadurch gekennzeichnet, dass sie am Stickstoff monosubstituierte oder nicht substituierte Iminiumsalze der Formeln

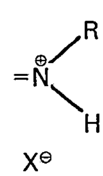 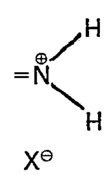

sind, in welchen R einen Substituenten und X ein Anion von Polybenzolsulfonsäure darstellen, wobei diese Iminiumsalze Additionsverbindungen

auf einer festen, starken Säure, ausgewählt unter den Polybenzolsulfonsäuren, eines reaktiven Imins, ausgewählt unter folgenden einfachen Iminen, sind:

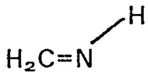 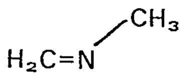

1 2

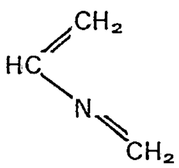 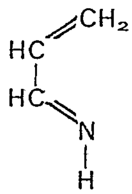 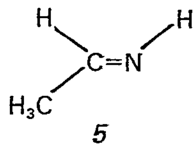

3 4 5

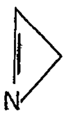 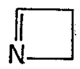 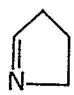 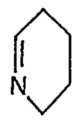

6 7 8 9

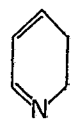 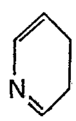

10 11

6. Als stabile Iminiumsalze nach Anspruch 5 das N-Methylmethaniminium-Polybenzolsulfonat und das Methaniminium-Polybenzolsulfonat.

7. Anwendung der Iminiumsalze nach Anspruch 5 oder 6, dadurch gekennzeichnet, dass man diese Iminiumsalze, die Synthone darstellen, Additionsrekationen mit Nucleophilen oder Zykloadditionsreaktionen mit olefinischen Verbindungen unterwirft, um Zwischenprodukte der Alkaloidsynthese und heterozyklische Verbindungen wie Alakloide des Typs Chinuclidin oder Isochinuclidin zu erhalten.

## Claims

1. Process for the stabilisation of reactive imines by transforming the said imines into iminium salts, characterised in that the said transformation is effected by an addition reaction of the imine in a dilute state on a solid strong acid with a large specific surface area chosen from polybezenesulphonic acids, the reaction, which leads to iminium salts monosubstituted or non-substituted on the nitrogen with the formulae:

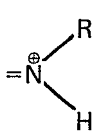 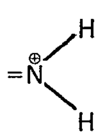

$X^{\ominus}$ $X^{\ominus}$

where R represents a substituent and X an anion of polybenzenesulphonic acid, being obtained by passing the imine in a vapour or gaseous phase, under vacuum, at normal temperature, over the solid strong acid, or by contacting the imine, at a low temperature, in the form of a dilute solution in an organic solvent with a low melting point, with the solid strong acid, and the said reactive imines, which comply with the following diagrammatic formulae:

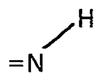 and 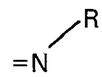

where R represents a subsituent, being chosen from the compounds with the following formulae 1 to 11:

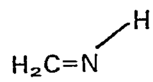 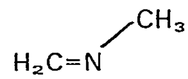

1 2

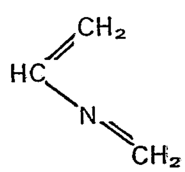 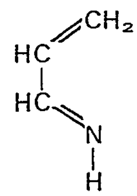 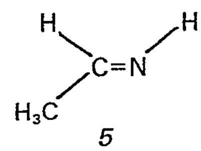

3 4 5

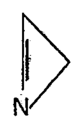 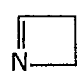 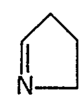 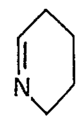

6 7 8 9

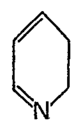 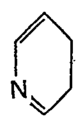

10 11

2. Process according to Claim 1, characterised in that the strong acid is in the form of porous grains or powder.

3. Process according to Claim 1 or 2, characterised in that the operation is carried out at −80° C when the imine is made to react at a low temperature in the form of a dilute solution.

4. Process according to any one of the Claims 1 to 3, characterised in that the imine used is obtained by dehydrohalogenation of the corresponding chloramine, and is directly transformed, without an intermediate isolation, into the required iminium salt.

5. Stable iminium salts such as obtained from the process according to any one of the Claims 1 to 4, characterised in that they are iminium salts monosubstituted or non-substituted on the nitrogen with the formulae:

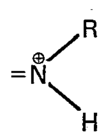 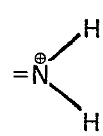

$X^{\ominus}$ $X^{\ominus}$

where R represents a substitutent and X an anion polybenzenesulphonic acid, the said iminium salts being addition compounds on a solid strong acid, chosen from polybenzenesulphonic acids, of a reactive imine chosen from the following simple imines:

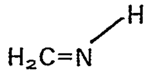

1

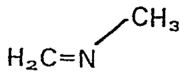

2

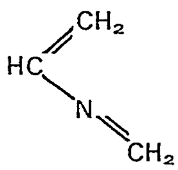

3

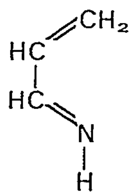

4

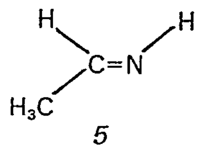

5

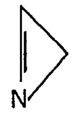

6

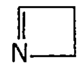

7

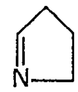

8

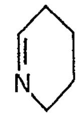

9

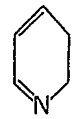

10

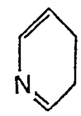

11

6. In respect of the stable iminium salts according to Claim 5, the polybenzenesulphate of N-methylmethaniminium and the polybenzene-sulphonate of methaniminium.

7. Application of the iminium salts according to Claim 5 or 6, characterised in that the said iminium salts, which are synthons, are subjected to addition reactions with nucleophiles or to cycloaddition reactions with olefin compounds, in order to obtain intermediates for alkaloid synthesis and heterocyclic compounds such as alkaloids of the quinuclidine or isoquinuclidine type.

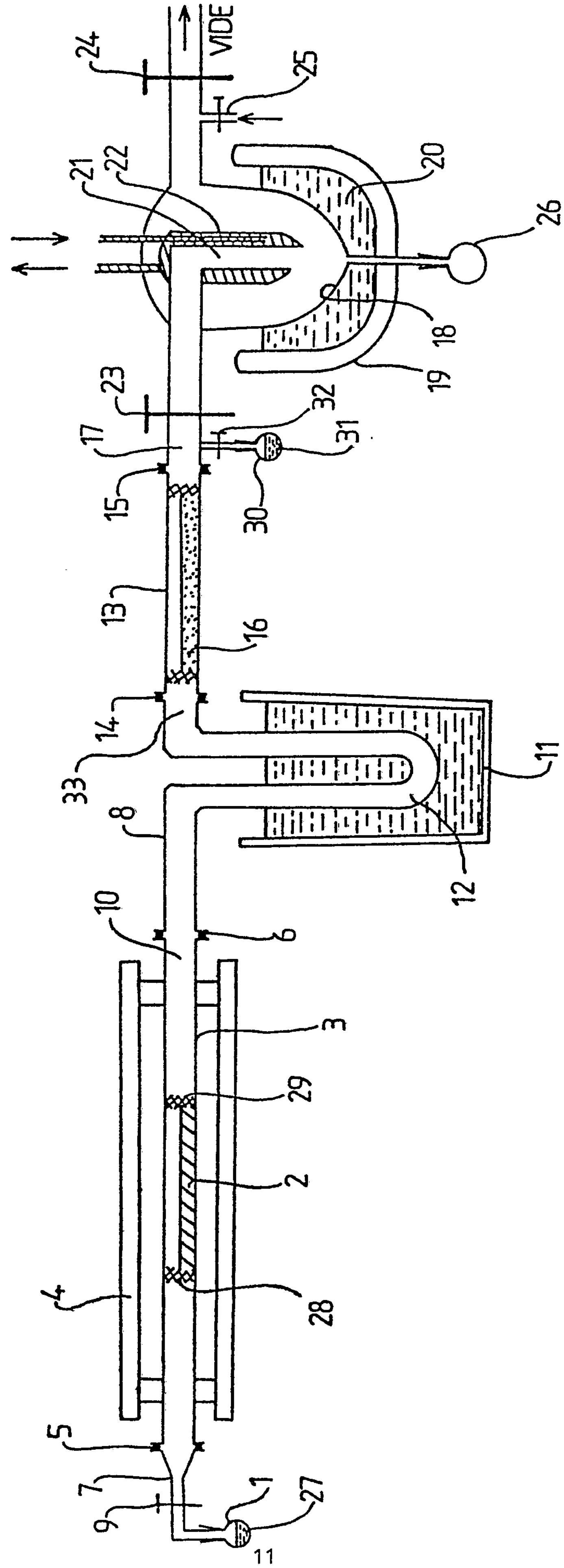
VIDE
24
25
21
22
20
26
18
19
23
32
31
17
15
30
13
16
14
33
8
11
12
10
6
3
29
2
28
4
5
7
1
27
9